# EUROPÄISCHE PATENTSCHRIFT

(11) **EP 1 032 316 B1**
(45) Veröffentlichungstag und Bekanntmachung des Hinweises auf die Patenterteilung: **24.03.2004**
(21) Anmeldenummer: 98958909.8
(22) Anmeldetag: 20.11.1998
(51) Int. Cl.: A61B 17/128, A61B 17/28

(54) **CLIPANLEGEGERÄT**
CLIP INSERTING TOOL
APPAREIL POUR LA POSE D'AGRAFES

(30) Priorität: 26.11.1997 DE 19752332
(43) Veröffentlichungstag der Anmeldung: 06.09.2000
(73) Patentinhaber: Aesculap AG & Co. KG, 78532 Tuttlingen (DE)
(72) Erfinder: KIENZLE, Karl-Ernst, D-78194 Immendingen (DE); MAYENBERGER, Rupert, D-78239 Rielasingen (DE); WEISSHAUPT, Dieter, D-78194 Immendingen (DE)
(74) Vertreter: Böhme, Ulrich, Dr. Dipl.-Phys.
(86) Internationale Anmeldenummer: PCT/EP1998/007478
(87) Internationale Veröffentlichungsnummer: WO 1999/026545

(56) Entgegenhaltungen:
- EP-A- 0 621 006
- WO-A-95/23557
- DE-C- 3 335 986
- US-A- 4 576 165
- US-A- 5 643 291

## Beschreibung

Die Erfindung betrifft ein Clipanlegegerät mit einem Griffteil und mit einem auf dieses aufsetzbaren, eine Anzahl von Clips enthaltenden Magazin und mit einem beweglichen Betätigungsgriff am Griffteil, der direkt oder indirekt mit einer Clipvorschiebeeinrichtung im Magazin in Eingriff steht, wenn das Magazin auf das Griffteil aufgesetzt ist.

Clipanlegegeräte dieser Art werden verwendet, um im Querschnitt U-förmige, elastisch aufbiegbare Clips an geeigneten Körperstellen zu applizieren, beispielsweise zur Unterbindung von Blutungen im Bereich der Kopfhaut. Die Clips werden dabei in dem Clipanlegegerät in einem Magazin zur Verfügung gestellt, in dem sich eine Anzahl von Clips befinden, die durch einen geeigneten Betätigungsmechanismus schrittweise im Magazin zu dessen offenem Ende vorgeschoben werden. Am offenen Ende befindet sich eine Öffnungs- und Anlegevorrichtung, die den jeweils vordersten Clip öffnet, an der gewünschten Stelle anlegt und dann freigibt, so daß der Clip durch die Eigenelastizität seiner Arme an der Anlegestelle gehalten wird.

Es ist bekannt, die Magazine als selbständige Baueinheiten auszugestalten, die nach Bedarf auf ein Griffteil aufgesetzt und nach Entleerung wieder von diesem abgenommen werden können. Das Griffteil weist Betätigungsvorrichtungen auf, die über eine mechanische Kupplung Vorschubglieder im Magazin betätigen, die ihrerseits die Clips im Magazin vorschieben (WO96/32891).

Beim Aufsetzen der Magazine auf derartige Griffteile ergibt sich das Problem, daß eine mechanische Kupplung zwischen dem Betätigungsgriff des Griffteils einerseits und dem Vorschubmechanismus im Magazin andererseits nur in einer ganz bestimmten Stellung des Betätigungsgriffs möglich ist. Andererseits besteht gerade beim Aufsetzen des Magazin die Gefahr, daß die Bedienungsperson den Betätigungsgriff unkontrolliert in die verschiedensten Positionen bewegt, und dann kann das Magazin nicht aufgesetzt werden.

Es ist Aufgabe der Erfindung, ein gattungsgemäßes Clipanlegegerät so auszugestalten, daß in jedem Fall beim Aufsetzen des Magazins auf das Griffteil die mechanische Kupplung des Betätigungsgriffs und der Clipvorschiebeeinrichtung im Magazin erfolgen kann.

Diese Aufgabe wird bei einem Clipanlegegerät der eingangs beschriebenen Art erfindungsgemäß dadurch gelöst, daß am Griffteil eine Verriegelungseinrichtung angeordnet ist, die in einer Verriegelungsposition den Betätigungsgriff in einer Eingriffsstellung verriegelt, in der beim Aufsetzen des Magazins der Eingriff mit der Clipvorschiebeeinrichtung herstellbar ist, und die den Betätigungsgriff in einer Freigabeposition freigibt.

Das Vorsehen einer solchen Verriegelungseinrichtung ermöglicht es, den Betätigungsgriff in der einzigen Stellung festzulegen, in der die mechanische Kupplung mit der Clipvorschiebeeinrichtung des Magazins möglich ist, und auf diese Weise ist sichergestellt, daß beim Aufsetzen des Magazins die Kupplung problemfrei erfolgen kann.

Besonders vorteilhaft ist es, wenn der Betätigungsgriff durch eine elastische Kraft in die Eingriffsstellung verschoben wird. Um die Verriegelungseinrichtung in die Verriegelungsposition verschieben zu können, ist es dann nicht mehr nötig, den Betätigungsgriff zunächst in diese Eingriffsstellung zu bewegen, diese Bewegung wird bereits durch die elastische Kraft verursacht, so daß sich der Betätigungsgriff nach dem Loslassen zwangsläufig in der Eingriffsstellung befindet. Durch das Anlegen der Verriegelungseinrichtung wird sichergestellt, daß diese Eingriffsstellung des Betätigungsgriffs auch dann beibehalten wird, wenn eine Bedienungsperson beim Aufsetzen des Magazins das Clipanlegegerät erfaßt und dabei gegebenenfalls Kräfte auf den Betätigungsgriff ausübt.

Die elastische Kraft zur Verschiebung des Betätigungsgriffs in die Eingriffsstellung kann beispielsweise durch eine zwischen Betätigungsgriff und Griffteil eingespannte Biegefeder erzeugt werden.

Es ist weiterhin besonders vorteilhaft, wenn die Verriegelungseinrichtung von dem aufgesetzten Magazin derart betätigbar ist, daß es sich dauerhaft in der Freigabeposition befindet. Das Magazin entriegelt also die Verriegelungseinrichtung, sobald es sich in der aufgesetzten Stellung befindet, so daß der Benutzer nach dem Aufsetzen des Magazins das Anlegegerät sofort benutzen kann, ohne vorher den Betätigungsgriff entriegeln zu müssen.

Gemäß einer besonders bevorzugten Ausführungsform ist dabei vorgesehen, daß die Verriegelungseinrichtung gegen die Wirkung einer elastischen Kraft aus der Verriegelungsposition in die Freigabeposition bewegbar ist. Während also das Magazin aufgesetzt ist, bleibt die Verriegelungseinrichtung dauerhaft in der Freigabeposition und ist damit unwirksam, ist das Magazin abgenommen wird dagegen automatisch eine Verriegelung erfolgen, da die Verriegelungseinrichtung unter der Wirkung der elastischen Kraft in die Verriegelungsposition gelangt, sobald sich der Betätigungsgriff in der Eingriffsstellung befindet.

Bei einer bevorzugten Ausführungsform kann vorgesehen sein, daß die Verriegelungseinrichtung einen Verriegelungskörper umfaßt, der unter der Wirkung einer Feder gegen den Betätigungsgriff oder ein von diesem verschiebbares Teil gedrückt wird und in der Eingriffsstellung des Betätigungsgriffs in eine Ausnehmung des Betätigungsgriffs oder hinter eine Kante des Betätigungsgriffs eintaucht.

Dabei ist es vorteilhaft, wenn der Verriegelungskörper in seiner Verriegelungsposition in die Bewegungsbahn des Magazins beim Aufsetzen auf das Griffteil hineinragt und wenn das Magazin eine Anlagefläche aufweist, die sich beim Aufsetzen des Magazins an den Vorsprung des Verriegelungskörpers anlegt und diesen dabei in die Freigabeposition verschiebt. Dabei wird also die Bewegung des Magazins beim Aufsetzen ausgenutzt, um die Verriegelungseinrichtung in die unwirksame Freigabeposition zu verschieben, der Benutzer muß also zur Entriegelung nicht gesondert tätig werden.

Insbesondere kann der Verriegelungskörper als in Längsrichtung verschiebbarer Stufenkolben ausgebildet sein, dessen dickerer Abschnitt in der Eingriffsstellung des Betätigungsgriffs in eine Ausnehmung des Betätigungsgriffs eintaucht, während der dünnere Abschnitt dann aus dem Griffteil hervorsteht.

Dabei kann der dünnere Abschnitt des Stufenkolbens durch einen Längsschlitz des Betätigungsgriffs hindurchragen, so daß der Betätigungsgriff bewegbar bleibt, auch wenn der Verriegelungskörper durch ihn hindurchragt.

Es ist günstig, wenn der dickere Abschnitt des Stufenkolbens in einem Gehäuse geführt gelagert ist, das eine stirnseitig an dem Stufenkolben anliegende Druckfeder aufnimmt.

Das Gehäuse kann lösbar am Griffteil gehalten sein, insbesondere durch Einschraubung.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines Clipanlegegeräts während des Aufsetzvorgangs des Magazins;

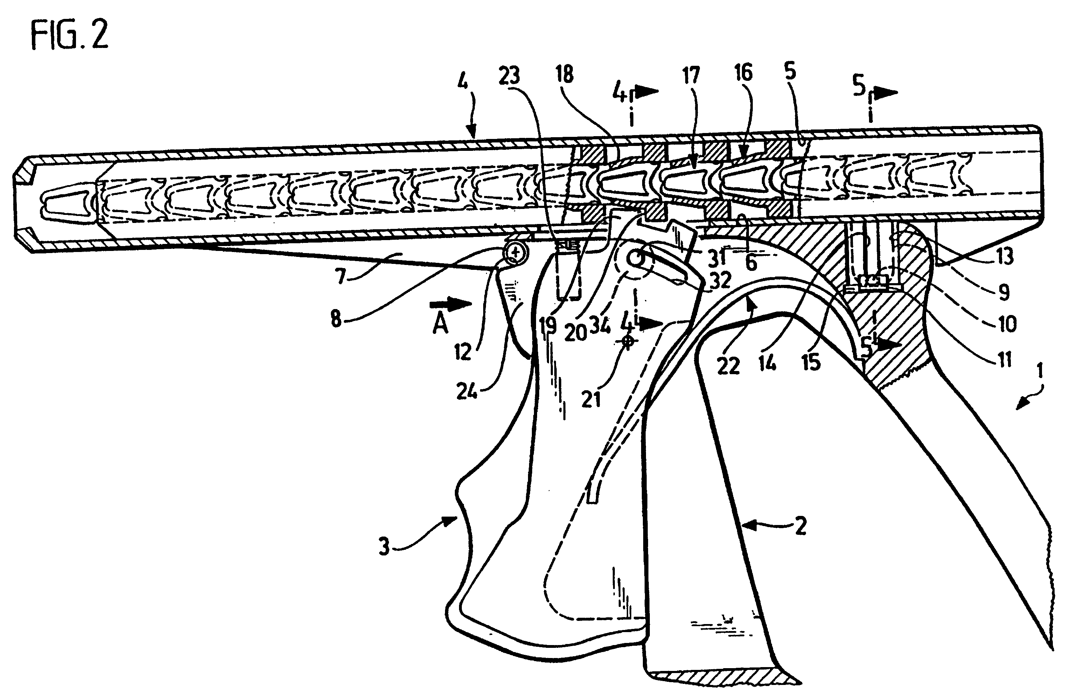

- Figur 2:: eine Schnittansicht längs Linie 2-2 in Figur 1;
- Figur 3:: eine Ansicht des Clipanlegegeräts in Richtung des Pfeils A in Figur 2;
- Figur 4:: eine Schnittansicht längs Linie 4-4 in Figur 2;
- Figur 5:: eine Schnittansicht längs Linie 5-5 in Figur 2 und
- Figur 6:: eine Schnittansicht längs Linie 6-6 in Figur 5.

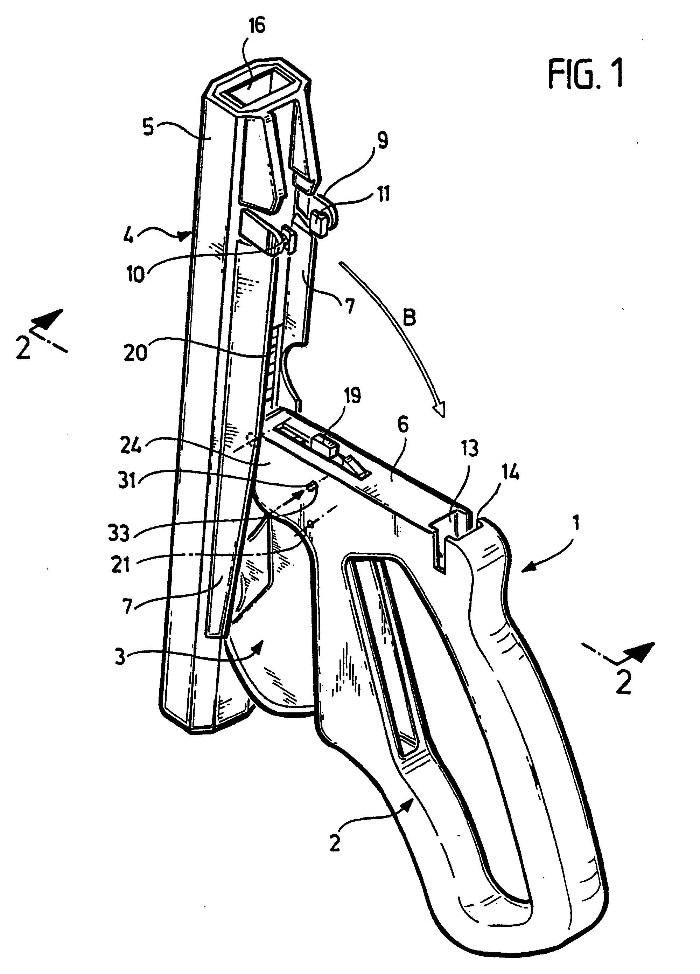

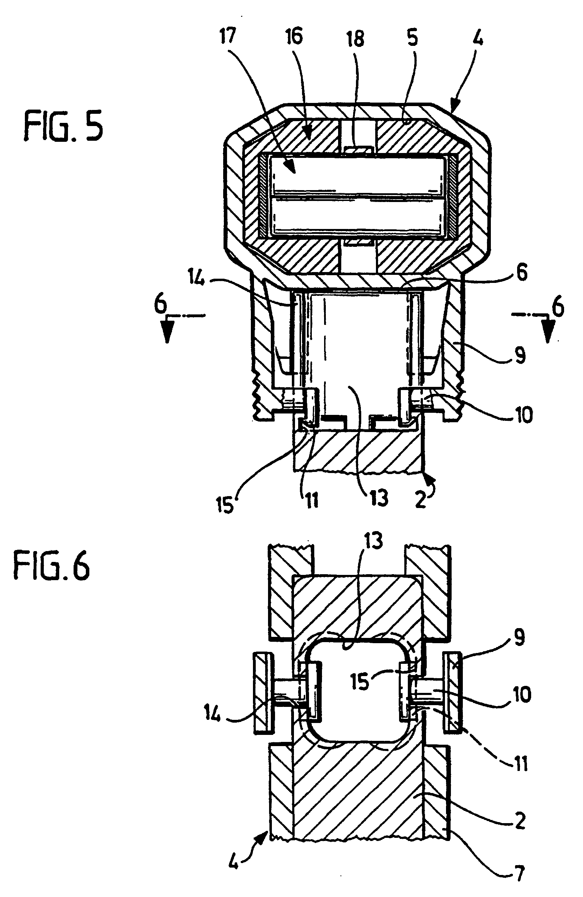

Das in der Zeichnung dargestellte Clipanlegegerät umfaßt ein pistolenförmiges Griffteil 1 mit einem feststehenden Handgriff 2 und einem schwenkbar am Griffteil 1 gelagerten Betätigungsgriff 3 sowie ein auf das Griffteil 1 aufsetzbares Magazin 4. Dieses Magazin 4 weist einen länglichen, zumindest an der Vorderseite offenen Schacht 5 auf und ist auf eine obere Auflagefläche 6 des Griffteils 1 auflegbar und in dieser Position mit dem Griffteil 1 verriegelbar. Zu diesem Zweck trägt der Schacht 5 an zwei parallelen, senkrecht nach unten abstehenden Wandteilen 7 nach innen abstehende Lagerzapfen 8 sowie ebenfalls nach unten abstehende Rastarme 9, die an ihren freien Enden nach innen abstehende Zapfen 10 mit nach unten und außen weisenden Rastvorsprüngen 11 tragen. Das Griffteil 1 weist an der Vorderseite der Auflagefläche 6 nach vorne offene, U-förmige Aufnahmen 12 auf, in die die Lagerzapfen 8 des Magazins 4 von vorne her eingeführt werden können, so daß dadurch eine Schwenkachse für das Magazin 4 ausgebildet wird. In dieser Position läßt sich das Magazin 4 in Richtung des Pfeils B in Figur 1 auf die Auflagefläche 6 verschwenken, wobei die Rastvorsprünge 11 des Magazins 4 in eine nach oben offene Rastausnehmung 13 am hinteren Ende der Auflagefläche 6 eintauchen. Die Rastausnehmung 13 weist an beiden Seiten senkrechte Schlitze 14 auf, durch die die Zapfen 10 von außen in die Rastausnehmung 13 eintreten können, und die Rastvorsprünge 11 hintergreifen Rücksprünge 15 am unteren Ende der Rastausnehmung 13, wenn das Magazin 4 vollständig auf die Auflagefläche 6 aufgelegt ist (Figur 5). Dadurch ist das Magazin 4 einmal über die in die Aufnahme 12 eingreifenden Zapfen 10 und zum anderen über die die Rücksprünge 15 hintergreifenden Rastvorsprünge 11 am Griffteil 1 gehalten.

Die Rastarme 9 sind durch einen Druck von außen elastisch gegeneinander verschwenkbar, und dies führt zu einem Ausschieben der Rastvorsprünge 11 aus den Rücksprüngen 15 und damit zu einer Freigabe des Magazins, das dann entgegen der Schwenkrichtung des Pfeils B verschwenkt und vom Griffteil 1 abgenommen werden kann.

Im Inneren des Magazins 4 ist ein kanalförmiges Gehäuse 16 längsverschieblich gelagert, das in seinem Innenraum eine Anzahl von gleichartigen Clips 17 aufnimmt, die hintereinander angeordnet sind. Die Clips 17 sind im Inneren des Gehäuses 16 in Längsrichtung verschieblich gelagert, werden jedoch durch an den Seiten der Clips angreifende Rückhalteelemente daran gehindert, im Gehäuse 16 zu dessen hinteren Ende hin verschoben zu werden. Diese Rückhalteelemente können beispielsweise elastisch in das Innere des Gehäuse 16 vorspringende Federzungen umfassen, die in der Zeichnung nicht gesondert dargestellt sind.

An der Oberseite und an der Unterseite des Gehäuses 16 sind ebenfalls schräg nach vorn in den Innenraum des Gehäuses 16 eintretende Federzungen 18 angeformt, und zwar etwa im Abstand der im Gehäuse 16 gehaltenen Clips 17, diese Federzungen 18 können an der Rückseite der Clips 17 anliegen und somit die Clips 17 zusammen mit dem Gehäuse 16 nach vorne schieben, wenn das Gehäuse 16 im Magazin 4 vorgeschoben wird. Wird das Gehäuse 16 anschließend wieder zurückgeschoben, bleiben die Clips 17 aber aufgrund der Rückhalteelemente in der vorgeschobenen Stellung stehen, so daß bei einer nachfolgenden Vorschubbewegung des Gehäuses 16 die Clips wiederum um die Vorschubstrecke des Gehäuses nach vorne geschoben werden. Durch abwechselndes Vor- und Zurückschieben des Gehäuses 16 ist es also möglich, die Clips 17 im Inneren des Magazins schrittweise zu dessen offenem Ende hinzuschieben.

Um eine solche reziprozierende Verschiebung des Gehäuses 16 im Magazin 4 hervorzurufen, greift ein mit dem Betätigungsgriff 3 verbundener Mitnehmer 19, der nach oben über die Auflagefläche 6 vorsteht, in eine Öffnung 20 an der Unterseite des Gehäuses 16 ein. Da die Schwenkachse 21 des Betätigungsgriffs 3 in einigem Abstand unterhalb der Auflagefläche 6 angeordnet ist, führt eine Verschwenkung des Betätigungsgriffs 3 zu einer Verschiebung des Mitnehmers 19 in Längsrichtung der Auflagefläche 6, und diese Längsverschiebung wird auf das Gehäuse 16 übertragen.

Am Handgriff 2 ist eine Biegefeder 22 gehalten, die sich mit ihrem freien Ende am Betätigungsgriff 3 abstützt und diesen in eine nach vorne ausgeschwenkte Stellung verschwenkt, in der der Mitnehmer 19 in einer am weitesten zurückgeschobenen Stellung steht. Diese Stellung wird nachfolgend als Eingriffsstellung bezeichnet. In dieser Eingriffsstellung wird der Betätigungsgriff 3 durch die Biegefeder 22 gegen einen Anschlag geschwenkt, der im dargestellten Ausführungsbeispiel als Schraube 23 ausgebildet ist und dessen Position durch mehr oder weniger tiefes Einschrauben dieser Schraube justierbar ist. Die Justierung erfolgt dabei so, daß in der Eingriffsstellung der Mitnehmer 19 problemlos in die Öffnung 20 des Gehäuses 16 eines Magazins 4 eingreift, wenn dieses Magazin 4 in der beschriebenen Weise auf das Griffteil 1 aufgesetzt wird. In jeder anderen Position des Mitnehmers 19 würde dieser gegen die Ränder der Öffnung 20 stoßen, in der Eingriffsstellung jedoch paßt der Mitnehmer 19 unmittelbar in die Öffnung 20 des Magazins, das im übrigen mit immer gleicher Stellung des Gehäuses 16 im Magazin 4 geliefert wird.

Der Handgriff 2 umgibt den Betätigungsgriff 3 im Bereich zwischen der Schwenkachse 21 und der Auflagefläche 6 beidseitig in Form einer am Betätigungsgriff 3 anliegenden Seitenwand 24. Eine der beiden Seitenwände weist eine durchgehende Gewindebohrung 25 auf, in die ein topfförmiges Gehäuse 26 eingeschraubt ist. Dieses Gehäuse 26 nimmt den dickeren Abschnitt 27 eines Stufenkolbens 28 auf, dieser dickere Abschnitt 27 ist in dem Gehäuse 26 längsverschieblich gelagert und wird durch eine Schraubenfeder 29, die sich einerseits am Boden 30 des Gehäuses und andererseits am dickeren Abschnitt 27 des Stufenkolbens 28 abstützt, aus dem Gehäuse 26 herausgedrängt gegen den Betätigungsgriff 3.

Ein sich an den dickeren Abschnitt 27 anschließender dünnerer, stabförmiger Abschnitt 31 durchsetzt einen Längsschlitz 32 im Betätigungsgriff 3 und eine Öffnung 33 in der gegenüberliegenden Seitenwand 24.

Auf der dem Gehäuse 26 zugewandten Seite weist der Betätigungsgriff 3 eine Vertiefung 34 auf, die mit dem Gehäuse 26 ausgerichtet ist, wenn der Betätigungsgriff 3 sich in der Eingriffsstellung befindet. In dieser Eingriffsstellung kann der Stufenkolben 28 mit dem dikkeren Abschnitt 27 unter der Wirkung der Schraubenfeder 29 in die Vertiefung 34 eingreifen und verriegelt somit den Betätigungsgriff 3 in der Eingriffsstellung. Wenn der dickere Abschnitt 27 des Stufenkolbens 28 in dieser Weise in der Vertiefung 34 eintaucht, ragt der dünnere Abschnitt 31 des Stufenkolbens 28 durch die Öffnung 33 hervor und steht dort geringfügig über.

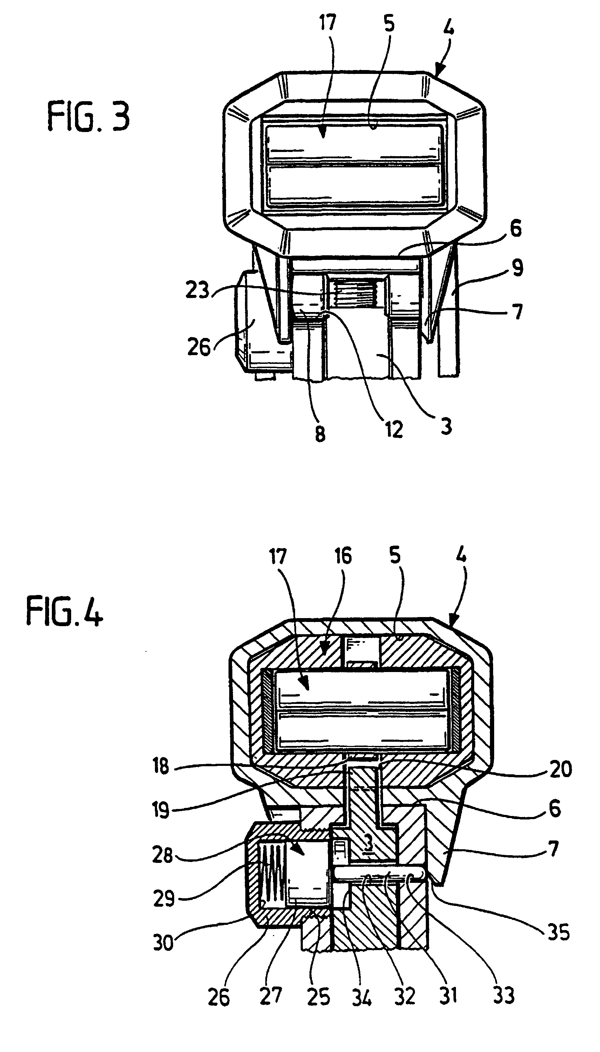

Das Wandteil 7 des Magazins 4 ist in dem Bereich, der der Öffnung 33 unmittelbar benachbart ist, als Aufgleitfläche 35 ausgebildet, die sich beim Aufsetzen des Magazins 4 an das aus der Öffnung 33 vorstehende Ende des dünneren Abschnitts 31 des Stufenkolbens 28 anlegt und beim vollständigen Aufsetzen des Magazins 4 diesen dünneren Abschnitt 31 in die Öffnung 33 vollständig einschiebt (Figur 4). Dadurch wird der Stufenkolben 28 entgegen der Wirkung der Schraubenfeder 29 in das Gehäuse 26 eingeschoben, und zwar so weit, daß der dickere Abschnitt 27 des Stufenkolbens 28 vollständig aus der Vertiefung 34 austritt. In dieser Stellung kann der Betätigungsgriff 3 verschwenkt werden, diese Stellung des Stufenkolbens 28 wird daher als Freigabeposition bezeichnet, während die Position, bei der der Stufenkolben 28 mit seinem dickeren Abschnitt 27 in die Vertiefung 34 eintaucht, als Verriegelungsposition zu bezeichnen ist, da dadurch ein Verschwenken des Betätigungsgriffs 3 verhindert wird.

Bei einem Griffteil 1, bei dem noch kein Magazin 4 aufgesetzt ist, wird der Handgriff 2 durch die Biegefeder 22 in die Eingriffsstellung verschwenkt, und in dieser Eingriffsstellung verschiebt die Schraubenfeder 29 den Stufenkolben 28 in die Verriegelungsposition, da in der Eingriffsstellung die Vertiefung 34 mit dem Gehäuse 26 ausgerichtet ist. Damit ist der Betätigungsgriff 3 gegen ein Verschwenken verriegelt.

Er befindet sich in dieser verriegelten Stellung in der Eingriffsstellung, so daß beim Aufsetzen des Magazins 4 sichergestellt ist, daß der Mitnehmer 19 in die dafür vorgesehene Öffnung 20 am Gehäuse 16 eintauchen kann. Im letzten Teil der Aufsetzbewegung des Magazins 4 legt sich die Aufgleitfläche 35 des Magazins an das freie Ende des dünneren Abschnitts 31 des Stufenkolbens 28 an und verschiebt diesen dadurch in die Freigabeposition, so daß nach dem Aufsetzen des Magazins 4 das Clipanlegegerät entriegelt ist und in der gewünschten Weise betätigt werden kann.

Sobald der Benutzer das Magazin nach dessen Leerung wieder abnimmt, erfolgt eine automatische Verriegelung, da der Handgriff 2 durch die Biegefeder in der beschriebenen Weise in die Eingriffsstellung verschwenkt und dort verriegelt wird.

## Patentansprüche

1. Clipanlegegerät mit einem Griffteil (1), mit einem auf dieses aufsetzbaren, eine Anzahl von Clips (17) enthaltenden Magazin (4) und mit einem beweglichen Betätigungsgriff (3) am Griffteil (1), der direkt oder indirekt mit einer Clipvorschiebeeinrichtung (16) im Magazin (4) in Eingriff steht, wenn das Magazin (4) auf das Griffteil (1) aufgesetzt ist, **dadurch gekennzeichnet, daß** am Griffteil (1) eine Verriegelungseinrichtung (28, 34) angeordnet ist, die in einer Verriegelungsposition den Betätigungsgriff (3) in einer Eingriffsstellung verriegelt, in der beim Aufsetzen des Magazins (4) der Eingriff mit der Clipvorschiebeeinrichtung (16) herstellbar ist, und die den Betätigungsgriff (3) in einer Freigabeposition freigibt.

2. Clipanlegegerät nach Anspruch 1, **dadurch gekennzeichnet, daß** der Betätigungsgriff (3) durch eine elastische Kraft in die Eingriffsstellung verschoben wird.

3. Clipanlegegerät nach Anspruch 2, **dadurch gekennzeichnet, daß** die elastische Kraft durch ein zwischen Betätigungsgriff (3) und Griffteil (2) eingespannte Biegefeder (22) erzeugt wird.

4. Clipanlegegerät nach einem der voranstehenden Ansprüche, **dadurch gekennzeichnet, daß** die Verriegelungseinrichtung (28, 34) von dem aufgesetzten Magazin (4) derart betätigbar ist, daß sie sich dauerhaft in Freigabeposition befindet.

5. Clipanlegegerät nach Anspruch 4, **dadurch gekennzeichnet, daß** die Verriegelungseinrichtung (28, 34) gegen die Wirkung einer elastischen Kraft (29) aus der Verriegelungsposition in die Freigabeposition bewegbar ist.

6. Clipanlegegerät nach einem der voranstehenden Ansprüche, **dadurch gekennzeichnet, daß** die Verriegelungseinrichtung einen Verriegelungskörper (28) umfaßt, der unter der Wirkung einer Feder (29) gegen den Betätigungsgriff (3) oder ein von diesem verschiebbares Teil gedrückt wird und in der Eingriffsstellung des Betätigungsgriffs in eine Ausnehmung (34) des Betätigungsgriffs oder hinter eine Kante des Betätigungsgriffs eintaucht.

7. Clipanlegegerät nach Anspruch 6, **dadurch gekennzeichnet, daß** der Verriegelungskörper (28) in seiner Verriegelungsposition in die Bewegungsbahn des Magazins (4) beim Aufsetzen auf das Griffteil (1) hineinragt und daß das Magazin (4) eine Anlagefläche (35) aufweist, die sich beim Aufsetzen des Magazins (4) an den Verriegelungskörper (28) anlegt und diesen dabei in die Freigabeposition verschiebt.

8. Clipanlegegerät nach Anspruch 7, **dadurch gekennzeichnet, daß** der Verriegelungskörper als in Längsrichtung verschiebbarer Stufenkolben (28) ausgebildet ist, dessen dickerer Abschnitt (27) in der Eingriffsstellung des Betätigungsgriffs (3) in die Ausnehmung (34) des Betätigungsgriffs (3) eintaucht, während der dünnere Abschnitt (31) dann aus dem Griffteil (2) hervorsteht.

9. Clipanlegegerät nach Anspruch 8, **dadurch gekennzeichnet, daß** der dünnere Abschnitt (31) des Stufenkolbens (28) durch einen Längsschlitz (32) des Betätigungsgriffs (3) hindurchragt.

10. Clipanlegegerät nach einem der Ansprüche 8 oder 9, **dadurch gekennzeichnet, daß** der dickere Abschnitt (27) des Stufenkolbens (28) in einem Gehäuse (26) geführt gelagert ist, das eine stirnseitig an dem Stufenkolben (28) anliegende Druckfeder (29) aufnimmt.

11. Clipanlegegerät nach Anspruch 10, **dadurch gekennzeichnet, daß** das Gehäuse (26) lösbar am Griffteil (2) gehalten ist.

12. Clipanlegegerät nach Anspruch 11, **dadurch gekennzeichnet, daß** das Gehäuse (26) in das Griffteil (2) eingeschraubt ist.

## Claims

1. A clip applicator comprising a handle part (1), a magazine (4) attachable thereto and containing a number of clips (17), and a movable operating handle (3) provided on the handle part (1) and in direct or indirect engagement with a clip feeder (16) in the magazine (4) when the magazine (4) is attached to the handle part (1), **characterised in that** a locking device (28, 34) is arranged on the handle part (1) and, in a locking position, locks the operating handle (3) in an engagement position in which it is engageable with the clip feeder (16) when the magazine (4) is attached, and releases the operating handle (3) in a release position.

2. A clip applicator according to claim 1, **characterised in that** the operating handle (3) is displaced into the engagement position by a resilient force.

3. A clip applicator according to claim 2, **characterised in that** the resilient force is generated by a bending spring (22) clamped between the operating handle (3) and a handle part (2).

4. A clip applicator according to any one of the preceding claims, **characterised in that** the locking device (28, 34) may be operated by the attached magazine (4) so that it is permanently in the release position.

5. A clip applicator according to claim 4, **characterised in that** the locking device (28, 34) is movable from the locking position into the release position against the action of a resilient force (29).

6. A clip applicator according to any one of the preceding claims, **characterised in that** the locking device comprises a locking member (28) which is pressed against the operating handle (3) or against a part displaceable by the operating handle (3) under the action of a spring (29) and which engages in a recess (34) of the operating handle or behind an edge of the operating handle when the latter is in the engagement position.

7. A clip applicator according to claim 6, **characterised in that** the locking member (28) projects, in its locking position, into the path of movement of the magazine (4) when the latter is attached to the handle part (1), and **in that** the magazine (4) has a contact surface (35) which comes to rest against the locking member (28) and, in so doing, displaces it into the release position when the magazine (4) is attached.

8. A clip applicator according to claim 7, **characterised in that** the locking member is formed as a longitudinally displaceable stepped piston (28), the thicker portion (27) of which engages in the recess (34) of the operating handle (3) when the latter is in the engagement position, while the thinner portion (31) then projects from the handle part (2).

9. A clip applicator according to claim 8, **characterised in that** the thinner portion (31) of the stepped piston (28) projects through a longitudinal slot (32) in the operating handle (3).

10. A clip applicator according to either one of claims 8 and 9, **characterised in that** the thicker portion (27) of the stepped piston (28) is mounted and guided in a housing (26) accommodating a compression spring (29) resting against the end of the stepped piston (28).

11. A clip applicator according to claim 10, **characterised in that** the housing (26) is detachably held on the handle part (2).

12. A clip applicator according to claim 11, **characterised in that** the housing (26) is screwed into the handle part (2).

## Revendications

1. Appareil de pose d'agrafes comportant une partie de préhension (1) comportant un magasin (4) pouvant être monté sur cette partie de préhension et contenant un nombre d'agrafes (17), et une poignée d'actionnement mobile (3) située sur l'organe de préhension (1) et qui engrène directement ou indirectement avec un dispositif (16) d'avance d'agrafes dans le magasin (4), lorsque le magasin (4) est monté sur la partie de préhension (1), **caractérisé en ce que** sur la partie de préhension (1) est disposé un dispositif de verrouillage (28,34), qui, dans une position de verrouillage, verrouille la poignée d'actionnement (3) dans une position d'engrènement, dans laquelle l'engrènement peut être établi avec le dispositif (16) d'avance des agrafes, lors de la mise en place du magasin (4), et qui libère la poignée d'actionnement (3) dans une position de libération.

2. Appareil de pose d'agrafes selon la revendication 1, **caractérisé en ce que** la poignée d'actionnement (3) est déplacée par une force élastique pour être amenée dans la position d'engrènement.

3. Appareil de pose d'agrafes selon la revendication 2, **caractérisé en ce que** la force élastique est produite par un ressort travaillant en flexion (22) qui est inséré entre la poignée d'actionnement (3) et la partie de préhension (2).

4. Appareil de pose d'agrafes selon l'une des revendications précédentes, **caractérisé en ce que** le dispositif de verrouillage (28,34) peut être actionné par le magasin monté, de telle sorte qu'il est situé en permanence dans la position de libération.

5. Appareil de pose d'agrafes selon la revendication 4, **caractérisé en ce que** le dispositif de verrouillage (28,34) est déplaçable à l'encontre de l'action d'une force élastique (29) depuis la position de verrouillage dans la position de libération.

6. Appareil de pose d'agrafes selon l'une des revendications précédentes, **caractérisé en ce que** le dispositif de verrouillage comprend un corps de verrouillage (28) qui, sous l'action d'un ressort (29) est repoussé contre la poignée d'actionnement (3) ou contre une partie déplaçable par cette poignée et pénètre dans un évidement (34) de la poignée d'actionnement ou en arrière d'un bord de la poignée d'actionnement lorsque la poignée d'actionnement est dans la position d'engrènement.

7. Appareil de pose d'agrafes selon la revendication 6, **caractérisé en ce que** dans sa position de verrouillage, le corps de verrouillage (28) pénètre dans la voie de déplacement du magasin (4) lors du montage sur la partie de préhension et que le magasin (4) possède une surface d'application (35) qui, lors du montage du magasin (4), s'applique contre le corps de verrouillage (28) et déplace ce dernier pour l'amener dans la position de libération.

8. Appareil de pose d'agrafes selon la revendication 7, **caractérisé en ce que** le corps de verrouillage est agencé sous la forme d'un piston étagé (28), qui est déplaçable dans la direction longitudinale et dont la section plus épaisse (27) pénètre dans l'évidement (34) de la poignée d'actionnement (3), lorsque cette dernière est dans la position d'engrènement, tandis que la section plus mince (31) fait alors saillie hors de la partie de préhension (2).

9. Appareil de pose d'agrafes selon la revendication 8, **caractérisé en ce que** la section plus mince (31) du piston étagé (28) pénètre dans une fente longitudinale (32) de la poignée d'actionnement (3).

10. Appareil de pose d'agrafes selon l'une des revendications 8 ou 9, **caractérisé en ce que** la section plus épaisse (27) du piston étagé (28) est montée de manière à être guidée dans un boîtier (26), qui loge un ressort de pression (29) qui s'applique frontalement contre le piston étagé (28).

11. Appareil de pose d'agrafes selon la revendication 10, **caractérisé en ce que** le boîtier (26) est retenu de façon amovible sur la partie de préhension (2).

12. Appareil de pose d'agrafes selon la revendication 11, **caractérisé en ce que** le boîtier (26) est vissé dans la partie de préhension (2).
